# EUROPEAN PATENT APPLICATION

(11) **EP 3 241 568 A1**
(43) Date of publication of application: **08.11.2017**
(21) Application number: 17159877.4
(22) Date of filing: 08.03.2017
(51) Int. Cl.: A61L 9/20, F25D 17/06

(54) **SYSTEM FOR INHIBITING THE GROWTH OF PATHOGENIC MICROORGANISMS AND STERILIZING DEVICE USED IN SUCH SYSTEM**

(30) Priority: 20.04.2016 BR 102016008926
(71) Applicant: Termotecnica Ltda., SC 89260-001 Joinville (BR)
(72) Inventor: Schmidt, Albano, SC 89203-072 Joinville (BR); Wei Hsin, Huang, SC 89216-275 Joinville (BR)
(74) Representative: Inchingalo, Simona

(57) **Abstract**

The present invention relates to a system for inhibiting the growth of pathogenic microorganisms, used in refrigerating appliances, air conditioners, and related apparatuses/equipment, using a sterilizing device (1) comprising an upper part (1A) and a lower part (1B), the upper part (1A) being provided with an electronic board (2) with ultraviolet LEDs (3) arranged in a duct (4), and the lower part (1B) having two parallel air circulation ducts (5), which are provided with a plurality of outlets (6), the device (1) being specially designed and located so that ultraviolet light directly impinges on the flow of cool air flowing inside the apparatus, causing the air circulating internally to have a sterilizing effect on the products stored inside a refrigerator or on the air produced by an air conditioner.

## Description

### Field of the Invention

The present invention relates to a system for inhibiting growth of pathogenic microorganisms, used in refrigeration appliances, air conditioners and related appliances/equipment, which uses a sterilizing device provided with an ultraviolet LED electronic board, especially designed and located so that ultraviolet light directly impinges on the flow of cold air flowing inside the apparatus, causing the air circulating internally to have a sterilizing effect on products stored inside a refrigerator or on the air produced by an air conditioner.

### Background of the Invention

To ensure the quality of food stored in a refrigerator or the quality of the air circulating in a closed environment, various treatments capable of decreasing the microbial load or even eliminating microorganisms have been developed.

The most used processes are generally of thermal origin, but in some cases non-thermal processes have been used. One of these process is irradiation with ultraviolet light.

The technology that uses ultraviolet light has been gaining ground in the segment of microorganism control because of its advantages over other sterilization devices, such as efficiency and reliability. Furthermore, disinfection by UV light does not introduce toxin into the environment, does not alter the chemical composition, taste or smell of food, causes minimal impact to the environment, among other benefits.

On the other hand, the main limitation of this technology is direct exposure to UV rays, since excessive doses of UV light can cause photo-oxidation with undesirable changes in the product sensory characteristics.

In refrigerators, the relatively low temperature can delay the bacteria-induced food deterioration. However, after a few days of storage, low temperature alone cannot prevent the deterioration of this foodstuff. Moreover, although a food item is still edible, the presence of bacteria can lead the user to have serious health problems.

Similarly, people who stay in a refrigerated environment for 24 hours a day, a few days in a row, may also have breathing problems because they breathe "stale" air, that is, contaminated air that is reused by the air conditioner.
Thereby, microorganisms dwelling in the environment are recirculating, considering a closed cycle of air circulation, keeping the air heavy and contaminated.

To overcome the above-mentioned problems, the prior art provides some systems that use UV light for inhibiting the growth of pathogenic microorganisms, applied in refrigerating appliances and air conditioners, among which we point out the following documents.

Document N°. US 2003/0019222 describes a refrigerator comprising a UV lamp, for irradiating ultraviolet rays through an air duct into a cold-storage compartment of the refrigerator; The invention further provides a light catalyst filter, installed in the air duct and activated by the ultraviolet rays; a fan for forming an air curtain when a door of the cold storage compartment of the refrigerator is opened; and a controlling device for lighting the UV lamp when the door of the cold storage compartment of the refrigerator is opened. In addition, the refrigerator can further comprise an operating device for setting or disabling an antimicrobial mode of the refrigerator; and a controlling device for lighting the UV lamp for a preset interval, and turning off the UV lamp when a door of the refrigerator is open, when the antimicrobial mode is disabled.

The refrigerator described in said document is an evolution when compared with the prior art, insofar as it uses an UV lamp system. However, it has some drawbacks, among which we stress the use of a UV light catalyst filter, requiring maintenance and periodic change thereof. Furthermore, the use of a UV emission lamp implies significant heat dissipation in the refrigerator internal environment, resulting in a higher thermal load to be removed by the cooling system of the appliance, resulting in greater energy consumption. Moreover, by promoting greater heat dissipation in the internal environment, it causes a greater variation of internal temperature, and, consequently, a lower conservation of the stored foodstuff, with loss of their organoleptic properties.

Another prior disclosure is document N°. US 2012/0085116, which describes a refrigerator with an ultraviolet LED comprising a freezer having an evaporator installed in the back of the freezer. A fan installed on the evaporator supplies cool air to the inside of the freezer and of the refrigerator. A base provided with ultraviolet LED is placed between a first member and a second member inside the freezer to enable the emission of a wave of ultraviolet light toward the interior space of the refrigerator, maintaining the sanitary conditions of the food stored in its interior.

The technique described in said document has the drawback that UV light directly affects the products that are stored inside the refrigerator, causing prolonged exposure of these products to the direct incidence of UV light, damaging the sensory characteristics thereof.

Another prior disclosure is document N°. US 2013/0104579, which relates to a refrigerator comprising a sealed compartment, provided with shelves and drawers, and a door disposed on a side of the sealed compartment, where foodstuff is stored, wherein the walls of the sealed compartment are coated with an ultraviolet-reflective material. The shelves and the drawer cover are provided with ultraviolet LEDs. An air circulation hub is also provided inside the sealed compartment. A fan supplies air to the air circulation hub making air flow inside a sealed compartment, radiating a wave of ultraviolet light.

Similarly, the technique described in said document has the drawback that UV light directly affects the products that are stored inside compartments and drawers, causing prolonged exposure of these products to the direct incidence of UV light, damaging the sensory characteristics thereof. Moreover, UV light covers only one refrigerator compartment, isolated from the others, restricting the application to the other internal areas of the appliance.

Thus, it is a prior art need to provide a system for inhibiting the growth of pathogenic microorganism in refrigerant appliances, air conditioners, and similar appliances/equipment, using a sterilizing device provided with an electronic board with ultraviolet LEDs, overcoming the aforementioned problems.

A simplified summary of the embodiments described in the instant invention will be described, such a summary being not an extensive general overview of all the embodiments contemplated herein. Moreover, it aims neither to identify key or critical elements nor to delineate the scope of such embodiments. Its sole purpose is that of providing some concepts of the simplified forms, as an introduction to the more detailed description that is provided below.

### Summary of the Invention

The present invention relates to a system for inhibiting the growth of pathogenic microorganism in refrigerant appliances, air conditioners, and similar appliances/equipment, using a sterilizing device provided with an electronic board with ultraviolet LEDs.

The operating principle of the system of the present invention is to permanently irradiating UV light on the cool air flow entering the apparatus, which then passes through the various points inside a refrigerator and/or the air that is produced by an air conditioner, recovering with it a portion of the microorganisms from the environment. The cool air flow returns to the passageway through UV light and is again subjected to the inhibitory action of UV light, providing sterilization of the environment.

The ultraviolet LED electronic board is placed on a surface of an air duct of the sterilizing device which is installed inside the refrigerator or air conditioner near the cold air flow inlet provided by the fan of the appliance. Thereby, the UV light directly affects the passing cool air flow, causing circulating cool air to have a sterilizing effect on the pathogenic microorganisms found in the environment.

The main advantage of the present invention is to provide a system for inhibiting the growth of pathogenic microorganisms inside refrigerators or in environments climatized by air conditioners by using a sterilizing device having a UV LED electronic board, in which UV light directly falls upon the flow of cool air circulating in the indoor environment.

Another advantage of the present invention is to prevent products stored inside a refrigerator from being exposed to the direct incidence of UV rays, preventing any effect of genetic deterioration of the foodstuff.

Another advantage of the present invention is to emit UV light through UV LEDs, in order to minimize the heat dissipation inside a refrigerator, thereby ensuring the maintenance of cool temperature in the environment.

Another advantage of the present invention is that it does not use UV light photocatalyst filtering element, preventing replacements or periodic maintenance thereof.

To achieve the aforementioned objectives and similar objectives, one or more embodiments include aspects that will be described below and specifically defined in the claims. The following description and the attached drawings show details of certain illustrative aspects of the disclosed embodiments. However, these aspects indicate only some of the many ways in which the principles of various embodiments can be used. Moreover, all the described embodiments are intended to include these aspects and their equivalents.

### Brief description of the figures

The characteristics, the nature and the advantages of the instant description will be more evident from the detailed description provided below, when read together with the drawings, in which the same references refer to the same elements, where:
Figure 1 - illustrates a schematic view of the system for inhibiting the growth of pathogenic microorganisms, subject matter of the present invention, inside a refrigerator;
Figure 2 - illustrates a perspective side view of the sterilizing device, subject matter of the instant invention;
Figure 3 - illustrates a partial view of the upper part of the sterilizing device;
Figure 4 - illustrates a perspective rear view of the sterilizing device, subject matter of the instant invention;
Figure 5 - illustrates a sectional view of the upper part of the sterilizing device;
Figure 6 - illustrates a schematic view, taken from the front part of the sterilizing device, illustrating the air flow;
Figure 7 - illustrates a schematic view, taken from the back part of the sterilizing device, illustrating the air flow;

### Detailed Description of the Invention

According to what the attached Figures illustrate, the present invention relates to a SYSTEM FOR INHIBITING THE GROWTH OF PATHOGENIC MICROORGANISMS, AND STERILIZING DEVICE USED IN SUCH SYSTEM, used in refrigeration appliances, air conditioners, and similar appliances/equipment, which, through an ultraviolet LED device, provides a sterilizing effect on products stored inside a refrigerator or on the air produced by an air conditioner.

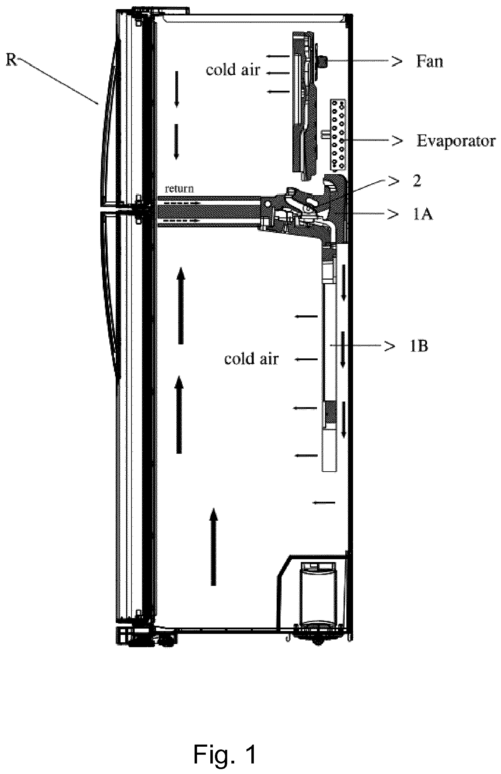

Reference will now be made to Figure 1, which refers to a schematic view of the preferred embodiment of the system of the present invention, illustrating the sterilizing air flow into the refrigerator, wherein the system comprises a sterilizing device (1) consisting of an upper part (1A) and a lower part (1B), wherein cool air enters through an opening (9) arranged in the front region of the upper part (1A), flows into a duct (4) provided with an electronic board (2) having UV LEDs (3) located in the upper part (1A), and then flows into two parallel air circulation ducts (5) arranged in the lower part (1B), which are provided with a plurality of outlets (6) whereby the already sterilized air is distributed into the refrigerator. The sterilizing device (1) is internally arranged in the middle upper part of a refrigerator (R), just below the air outlet provided by the refrigerator fan.

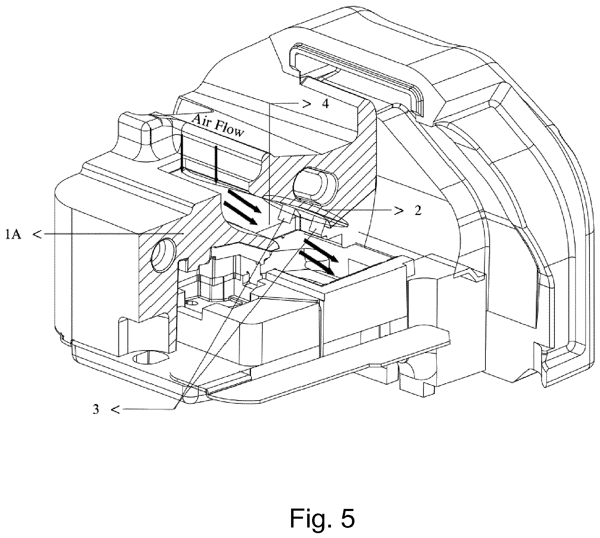

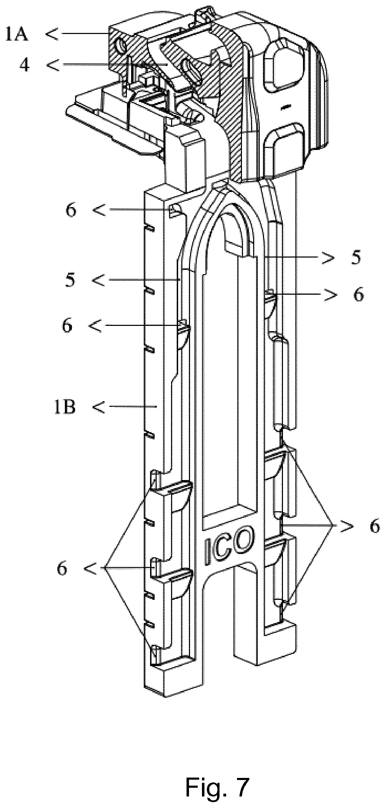

The electronic board (2) with the UV LEDs (3) is strategically placed in the duct (4) of the upper part (1A) of the device (1), as illustrated in Figure 5, so that the efficiency of the incidence of the UV LED light is as high as possible, considering that the air is integrally channeled into the duct (4) to flow under the total incidence of light towards the ducts (5) of the lower part (1B), and then, the air is distributed to all the inner compartment of the refrigerator(6), as illustrated in Figure 7, for proper cooling of the items stored in shelves and drawers, with sterilizing properties.

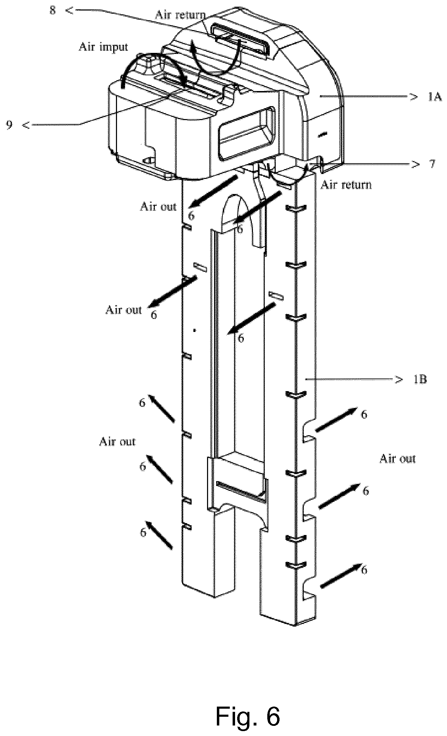

The sterilizing cool air flow cycle is repeated when the door of the refrigerator (R) remains closed, returning through each of the inlets (7) arranged in the lower side of the upper part (1A), flowing out through an opening(8) arranged in the upper central region, then, flowing in through the opening(9) in the front region, both openings (8, 9) being arranged in the upper part (1A), to circulate again through the duct (4), flowing through the UV LEDs(3), flowing into the ducts (5) and out through the outlets (6) of the lower part (1B) of the device (1), as illustrated in Figure 6.

By traversing the various points in the inner compartment of the refrigerator, the sterilizing cool air rescues a portion of the microorganisms from the site. As the sterilizing cool air flow cycle repeats, the sterilizing effect increases and the presence of pathogenic microorganisms decreases considerably in the environment.

In the system of the present invention, the incidence of the UV LED (3) light inside the tube (4) occurs in the airflow upstream of the internal compartment of the refrigerator. Thereby, air free of pathogenic microorganisms is blown into the internal environment of the refrigerator.

The efficiency of the sterilizing action of the air flow through the ducts (4, 5) of the device (1) is favored by having a condition of air temperature around 5°C inside the refrigerator (R).

When the door of the refrigerator (R) is opened, a new mass of air and microorganisms enters the refrigerator. Thereby, this new mass of air returns its cycle through the duct (4), flows through the UV LEDs (3), flows into the ducts (5) and flows out the outlets (6) of the lower part (1B) of the sterilizing device.

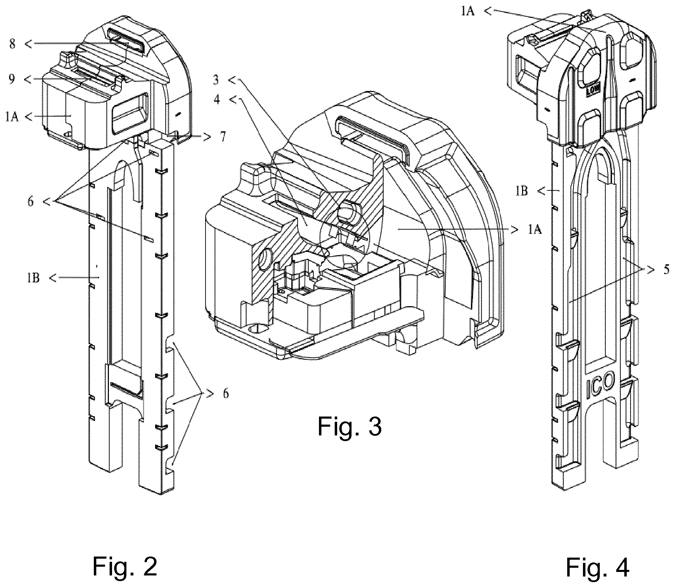

Reference is now made to Figures 2, 3 and 4, which illustrate the sterilizing device (1) of the present invention, consisting of an upper part (1A) and a lower part (1B), the upper part (1A) being provided with an electronic plate (2) having UV LEDs (3) disposed in an air circulation duct in an enclosed and compact site, further comprising two inlets (7) arranged one on each lower side, an opening (8) arranged in the upper central region, and an opening (9) in the front region, the lower part (1B) having two parallel air circulation ducts (5) which are provided with a plurality of outlets (6) through which the already sterilized air is distributed inside the refrigerator. The sterilizing device (1) is internally arranged in the middle upper part of the refrigerator (R), just below the air outlet provided by the fan of the refrigerator itself.

The electronic board (2) has the function of connecting the UV LEDs (3) to an electric power supply, for instance, the power supply of the refrigerator (R) itself. Preferably, the UV LEDs (3) are permanently connected, as default mode, considering that the refrigerator is always operating with the door closed. If the refrigerator door opens, there is usually a command on the refrigerator control board to turn off the fan, and therefore, forced convection stops so that less cool air escapes, and thus sterilized air out.

In an alternative embodiment, the system in conjunction with the sterilizing device of the present invention may be applied to an air conditioner with the same efficiency and reliability.

A person skilled in the art would easily understand that the invention can undergo modifications without thereby departing from the concepts set forth in the foregoing description. These modifications must be considered as included within the scope of the invention. Accordingly, the particular embodiments described in detail above are merely illustrative and not restrictive as to the scope of the invention, thereto the full extension of the appended claims and any and all equivalents thereof must be given.

## Claims

1. A system for inhibiting the growth of pathogenic microorganisms, **CHARACTERIZED in that** it comprises a sterilizing device (1) arranged in the refrigerator (R), just below the upstream cool air flow (R) supplied by the fan of the refrigerator (R), the sterilizing device (1) consisting of an upper part (1A) and a lower part (1B), wherein
the upper part (1A) comprises an electronic board (2) provided with UV LEDs (3) arranged in an enclosure and compact site of an air circulation duct (4), and it also has two inlets (7) arranged one on each lower side, an opening (8) arranged in the upper central region, and an opening (9) in the front region; and
the lower part (1B) comprises two parallel air circulation ducts (5), which are provided with a plurality of outlets (6);
wherein cool air flows in through the opening (9) and is integrally channeled into the duct (4) to flow under the total incidence of the UV LED light (3) towards the ducts (5) of the lower part (1B), and then, flow out already sterilized through the outlets (6) so that the sterilizing air circulates in the internal environment of the refrigerator.

2. The system, according to claim 1, **CHARACTERIZED in that** the sterilizing air flow cycle is repeated so that the mass of air returns through each of the inlets (7), flows out through the opening (8) and flows in the opening (9), and then again flows through the duct (4), passes through the UV LEDs (3), flows into the ducts (5) and flows out through the outlets (6) of the sterilizing device (1).

3. The system, according to claim 1, **CHARACTERIZED in that** the UV LEDs (3) are permanently switched on as default mode when the refrigerator door (R) is closed.

4. A sterilizing device (1), **CHARACTERIZED in that** it consists of an upper part (1A) and a lower part (1B), wherein the upper part (1A) comprises an electronic board (2) provided with UV LEDs (3) arranged in the air circulation duct (4), and it also has two inlets (7) arranged one on each lower side, an opening (8) arranged in the upper central region, and an opening (9) in the front region; and
the lower part (1B) has two air parallel circulation ducts (5), which are provided with a plurality of outlets (6), wherein the device (1) is arranged in the refrigerator (R), just below the upstream cool air flow supplied by the fan of the refrigerator (R).

5. The sterilizing device (1), according to claim 4, **CHARACTERIZED in that** the electronic board (2) connects the UV LEDs (3) with a power supply of the refrigerator (R).
